# NEW EUROPEAN PATENT SPECIFICATION

(11) **EP 3 021 882 B2**
(45) Date of publication and mention of the opposition decision: **27.01.2021**
(45) Mention of the grant of the patent: 18.04.2018
(21) Application number: 14748027.1
(22) Date of filing: 14.07.2014
(51) Int. Cl.: A61L 27/34, A61L 29/08, A61L 31/10, A61L 27/50, A61L 29/14, A61L 31/14

(54) **LUBRICIOUS COATING COMPOSITIONS**
GLEITFÄHIGE BESCHICHTUNGSZUSAMMENSETZUNGEN
COMPOSITIONS DE REVÊTEMENT LUBRIFIANT

(30) Priority: 15.07.2013 US 201361846376 P
(43) Date of publication of application: 25.05.2016
(73) Proprietor: Boston Scientific Scimed, Inc., Maple Grove, MN 55311-1566 (US)
(72) Inventor: ZENG, Hongxia, Maple Grove, Minnesota 55311 (US); SEPPALA, Jan, Maple Grove, Minnesota 55369 (US); CHEN, Yen-Lane, New Brighton, Minnesota 55311 (US); GOEL, Raghav, New Delhi 110096 (IN)
(74) Representative: Vossius & Partner Patentanwälte Rechtsanwälte mbB
(86) International application number: PCT/US2014/046534
(87) International publication number: WO 2015/009625

(56) References cited:
- WO-A1-00/61205
- WO-A1-2004/031253
- US-A1- 2007 003 707
- US-A1- 2013 123 664

## Description

### BACKGROUND

There is a desire in the medical arts to provide a variety of medical articles with increased lubricity relative to the materials commonly employed in their manufacture. This is particularly desirable, for instance, when the medical articles are medical devices to be implanted or inserted into the body. For example, as minimally invasive surgical techniques have improved, it has become increasingly common to insert and retrieve medical devices through catheters and the like having considerable length. Accordingly, it is desirable to minimize friction between the catheters that carry such devices and the devices themselves as well as with tissue with which they may come in contact. In the past, the industry has employed various hydrophobic oils and coatings such as olive oil, silicone, and the like as lubricants. Hydrophilic coatings, particularly hydrogens, also have been employed to impart lubricity to a variety of medical devices.

### SUMMARY OF THE INVENTION

In one aspect, the present invention pertains to lubricous coating compositions as defined in the annexed claims that comprise (a) a higher molecular weight polyvinylpyrrolidone (also referred to herein as higher MW PVP), (b) a lower molecular weight polyvinylpyrrolidone (also referred to herein as lower MW PVP), and (c) a polyfunctional unsaturated crosslinking agent.

In some embodiments, such lubricous coating compositions are present in crosslinked form on the surface of a medical article.

In some embodiments, the lubricous coating compositions further comprise a solvent. Such compositions may be, for example, applied to a substrate in the form of a layer and subsequently crosslinked, thereby forming a lubricious coating on the substrate.

In another aspect, the present invention is directed to methods for forming various medical articles.

An advantage of the present invention is that coatings can be provided which are both lubricious and durable. Another advantage of the present invention is that coatings can be provided which produce very low levels of particulates when in contact with aqueous fluids.

These and other aspects, embodiments and advantages of the present invention will become immediately apparent to those of ordinary skill in the art upon review of the Detailed Description and claims to follow.

### BRIEF DESCRIPTION OF THE DRAWINGS

Fig. 1 is a plot of frictional force (g) vs. number of friction cycles for different grades of PVP.
Fig. 2 is a plot of frictional force (g) vs. number of friction cycles for different ratios of higher MW PVP to lower MW PVP.
Fig. 3 is a plot of relative particle count (>10 micrometer) as a function of different ratios of higher MW PVP to lower MW PVP.
Fig. 4 is a plot of relative particle count (>50 micrometer) as a function of different ratios of higher MW PVP to lower MW PVP.
Fig. 5 is a plot of frictional force (g) vs. number of friction cycles for different overall solids content.
Fig. 6 is a plot of relative particle count (>10 micrometer) as a function of overall solids content.
Fig. 7 is a plot of relative particle count (>50 micrometer) as a function of overall solids content.
Fig. 8 is a plot of frictional force (g) vs. number of friction cycles for different ratios of PVP to NPGDA.
Fig. 9 is a plot of relative particle count (>50 micrometer) as a function of different ratios of PVP to NPGDA.

### DETAILED DESCRIPTION OF THE INVENTION

A more complete understanding of the present invention is available by reference to the following detailed description of numerous aspects and embodiments of the invention. The detailed description of the invention which follows is intended to illustrate but not limit the invention.

In one aspect, the present disclosure pertains to lubricous coating compositions for various articles. The compositions comprise a mixture of a higher molecular weight polyvinylpyrrolidone (higher MW PVP) and a lower molecular weight polyvinylpyrrolidone (lower MW PVP) as well as a crosslinking agent. The weight ratio of higher molecular weight polyvinylpyrrolidone to lower molecular weight polyvinylpyrrolidone ranges from 70:30 to 90:10.

Because it is difficult to measure molecular weight of a sample of PVP directly, the K-value has been adopted to classify the molecular weight of PVP. The K-value is a function of the average degree of polymerization and intrinsic viscosity of a given polymer and is calculated from the kinematic viscosity of a 1% w/v aqueous solution of the polymer.

As used herein, a higher MW PVP is defined as one having a K-value between 60 and 95 (e.g., ranging from 60 to 65 to 70 to 75 to 80 to 85 to 90 to 95). This corresponds to a weight average molecular weight between approximately 100,000 and 1,300,000. In preferred embodiments, the higher MW PVP will have a K-value between 85 and 95.

As used herein, a lower MW PVP is defined as one having a K-value between 5 and 35 (e.g., ranging from 5 to 10 to 15 to 20 to 25 to 30 to 35). This corresponds to a weight average molecular weight between approximately 2,000 and 95,000. In preferred embodiments, the lower MW PVP will have a K-value between 15 and 32.

Examples of polyvinylpyrrolidone materials useful in the present disclosure include Povidone K12, Povidone K15, Povidone K17, Povidone K25, Povidone K30, Povidone K60, Povidone K90 and Povidone K120, among others. Polyvinylpyrrolidone is available from BASF Corp, Parsippany, NJ, USA under the tradenames Kollidon® and Luvitec® and from Ashland Inc., Halethorpe, MD, USA as Plasdone®.

Depending on the composition, the crosslinking agent may be substantially uncrosslinked (degree of crosslinking less than 5%), partially crosslinked (degree of crosslinking between 5% and 95%), or substantially completely crosslinked (degree of crosslinking greater than 99%. The degree of crosslinking may range from 1% or less to 2% to 5% to 10% to 25% to 50% to 75% to 90% to 95% to 98% to 99% or more.

In some embodiments, the lubricous coating compositions are present on the surface of a medical article in solid form, with the crosslinking agent being at least partially crosslinked.

In some embodiments, the lubricous coating compositions are present in liquid form, with the crosslinking agent being substantially uncrosslinked. Such compositions may be, for example, applied to a substrate in the form of a layer and then at least partially crosslinked, thereby forming a lubricious coating on the substrate. In some embodiments, such compositions comprise a solvent, which may be removed before, during and/or after a crosslinking step.

Polyfunctional ethylenically unsaturated monomers are present as crosslinking agents. The polyfunctional ethylenically unsaturated monomers are compounds, including monomeric and oligomeric compounds, that have two or more ethylenically unsaturated groups thereon that may be readily polymerized by a radical mechanism to form a polymer. Typically, such compounds have a number average of about 5000 or less, more typically about 1000 or less. Suitable polyfunctional ethylenically unsaturated monomers include di- and tri-functional acrylate and methacrylate compounds, collectively referred to as (meth)acrylate compounds, including (meth)acrylate esters, as well as divinyl and trivinyl compounds. Specific examples of polyfunctional ethylenically unsaturated monomers that may be used in the compositions of the present disclosure include neopentyl glycol di(meth)acrylates, including neopentyl glycol diacrylate (NPGDA), ethylene glycol di(meth)acrylates, 1,3-propylene glycol di(meth)acrylates, 1,4-butanediol di(meth)acrylates, 1,6-hexanediol di(meth)acrylates, diethylene glycol di(meth)acrylates, triethylene glycol di(meth)acrylates, tetra ethylene glycol di(meth)acrylates, and polyethylene glycol di(meth)acrylates. In some embodiments the ethylenically unsaturated monomers that may be employed are alkoxylated and include ethoxylated and propoxylated (meth)acrylates.

Without being bound by theory, it is believed that the polyfunctional ethylenically unsaturated monomer(s) of the crosslinking agent are crosslinked upon exposure heat or actinic radiation such as UV irradiation, whereupon the crosslinked polyfunctional ethylenically unsaturated monomer(s) acts like a mesh that holds at least a portion of the PVP in place (such a system is known as a semi-interpenetrating polymer network).

The weight ratio of total polyvinylpyrrolidone to polyfunctional ethylenically unsaturated monomer(s) is in the range of from 0.5:1 to 4:1, preferably 1:1 to 2:1.

Monofunctional ethylenically unsaturated monomers may also be optionally included in some compositions (and thus excluded in other compositions). Examples include mono(meth)acrylate esters, mono-vinyl compounds, and so forth.

An additional hydrophilic polymer may also be optionally included in some compositions (and thus excluded in other compositions). Examples of such polymers, which may be included (or excluded), include polyethylene glycol, polypropylene glycol, polyvinylpryrrolidone, hydrophilic urethane polymers, including acrylated urethanes, and so forth.

A free radical initiator may also be optionally included in some compositions of the present disclosure (and thus excluded in other compositions). The free radical initiator may be, for example, a photoinitiator. Non-limiting examples of free radical photoinitiators that may be employed include benzophenones, ketones, acrylated amine synergists, alpha-amino ketones, acyl phosphine oxides including bis-acyl phosphine oxides, and benzil ketals. More specific examples of photoinitiators suitable for use herein include, but are not limited to, 2-phenyl-1-indanone; IRGACURE 184 from Ciba Specialty Chemicals, BENACURE 184 from Mayzo and SARCURE SR1122 from Sartomer, all of which are 1-hydroxylcyclohexylphenyl ketone (HCPK) initiators; BENACURE BP benzophenone; BENACURE 651 and IRGACURE 651, both of which are benzil dimethyl ketal or 2,2'dimethoxy-2-phenylacetophenone; BENACURE 1732 hydroxy-2-methyl-1-phenyl-1-propanone; IRGACURE 819 bis(2,4,6-trimethylbenzoyl)-phenylphosphineoxide, IRGACURE 907 2-Methyl-1-[4-(methylthio)phenyl]-2-(4-morpholinyl)-1-propanone; IRGACURE 369 morpholinoketone; and so forth and blends thereof. Photoinitiators are also available commercially in a variety of blends. Examples of commercially available blends include, but are not limited to, SARCURE SR1136 is a blend of 4-methylbenzophenone and benzophenone; SARCURE SR1137 is a blend of trimethylbenzophenone and methylbenzophenone; and BENACURE 500, a blend of 1-hydroxylcyclohexylphenyl ketone and benzophenone.

Other optional additives may be employed in the coating compositions of the present disclosure including, but not limited to, flow or viscosity modifiers, antioxidants, coupling agents, surfactants, therapeutic agents, and so forth. Any such additives are typically incorporated into the composition at levels of 10% or less (e.g., ranging from 10% to 5% to 2% to 1% to 0.5% or less), based on the dry weight (i.e. excluding solvent) of the composition.

In preparing liquid coating compositions for application to a substrate, the higher and lower molecular weight polyvinylpyrrolidones are suitably mixed with polyfunctional unsaturated crosslinking agent in a solvent that contains one or more solvent species. Examples of suitable solvents species include, but are not limited to, water and organic solvents including lower alcohols such as methanol, ethanol, and isopropyl alcohol (IPA), linear or cyclic carboxamides such as N,N-dimethylacetamide (DMAC), N,N-diethylacetamide, dimethylformamide (DMF), ethyl formamide, diethylformamide, N-methyl-2-pyrrolidone (NMP); dimethyl sulfoxide (DMSO), acetonitrile, acetone and acetyl acetone, acrylonitrile, benzonitriledimethyl acetamide, 1,4-dioxane, dipropyl sulfone, aromatic solvents such as toluene and xylene, nitrobenzene, phenylacetate, propionitrile, and so forth. Preferred solvent species are water soluble. Blends of solvent species such as those set forth above may be used. In one preferred embodiment, isopropyl alcohol in combination with water acts as a suitable solvent. In many embodiments, the ratio of water to alcohol ranges from 0:100 to 50:50, more typically 10:90 to 50:50.

Typically, the liquid coating compositions for use in the present disclosure will contain from 1% to 5% solids, more typically from 2 to 4% solids.

The mixture of solvent and coating composition may be applied to the medical device by any method known in the art including, but not limited to, spraying, dipping, rolling, painting (e.g., brush painting, sponge painting, etc.), and so forth. The coating may then be allowed to dry, by evaporation of the solvent. The solvent may be more readily evaporated at an elevated temperature, although room temperature drying is typically acceptable.

A variety of substrate materials may be used in conjunction with the present disclosure including organic and inorganic substrates, typically polymer substrates, metal substrates and glass substrates, among others. Examples of metal substrates include pure metals such as platinum, gold, iridium and titanium, or and metal alloys such as stainless steel including platinum enriched stainless steel (PERSS), Nitinol alloys, and cobalt chromium alloys.

Examples of polymer substrates include the following, among many others: (a) olefin homopolymers and copolymers, including homopolymers and copolymers of C2-C8 alkenes, for example, polyethylene and polypropylene, ethylene -vinyl acetate copolymers (EVA), and isobutylene-styrene copolymers, including block copolymers comprising one or more polystyrene blocks and one or more polyisobutylene blocks, for instance, poly(styrene-*b-*isobutylene-*b*-styrene) (SIBS), among others, (b) polyamides such as nylons , polyether-polyamide block copolymers such as poly(tetramethylene oxide-*b*-polyamide-12) block copolymer, available from Elf Atochem as PEBAX, among others, (c) fluoropolymers, including homopolymers and copolymers of C2-C8 alkenes in which one or more hydrogen atoms are substituted with fluorine, for example, polytetrafluoroethylene (PTFE), polyhexafluoropropene (PVDF), polyvinylidene fluoride (PVDF), and poly(vinylidene fluoride-*co*-hexafluoropropene) (PVDF-HFP), among others, (d) polyurethane copolymers, including copolymers that are polyether based, polyester based, polycarbonate based, aromatic based and aliphatic based, including polyisobutylene based polyurethanes (PIB-PU), among others, and (e) silicone homopolymers and copolymers (also referred to as polysiloxanes) such as polydimethylsiloxane.

Examples of substrates include medical article substrates, specific example of which include medical device substrates, for instance, implantable or insertable medical device substrates. A variety of devices may thus be partially or completely coated with compositions in accordance with the present disclosure, including, for example, catheters (e.g., renal or vascular catheters), balloons, catheter shafts, guide wires, filters (e.g., vena cava filters), stents (including coronary vascular stents, cerebral stents, urethral stents, ureteral stents, biliary stents, tracheal stents, gastrointestinal stents and esophageal stents), stent grafts, cerebral aneurysm filler coils (including Guglilmi detachable coils and metal coils), vascular grafts, myocardial plugs, patches, pacemakers and pacemaker leads, heart valves, vascular valves, tissue engineering scaffolds for cartilage, bone, skin and other in vivo tissue regeneration, and so forth.

Typically no primer layer or coupling agent is applied to the substrate before the coating is applied. However, in some embodiments, the substrate may be treated with plasma or corona discharge before application of the coating composition.

Coating compositions in accordance with the present disclosure may be cured, for example, by exposing the coating composition to heat or actinic radiation such as UV light for a short period of time. This initiates polymerization/crosslinking of the ethylenically unsaturated monomer(s). The polyfunctionality of at least some of the ethylenically unsaturated monomer(s) produces a high degree of crosslinking upon polymerization. At least for compositions based on acrylate esters it is generally desirable to cure in a low oxygen atmosphere, such as under a blanket of nitrogen, helium or argon gas. The amount of time needed to cure the surface is dependent on the source of energy, the relative amounts of constituents in the composition, the thickness of the coating desired, and other factors. Generally, the amount of time required for thermal cure is from about 1 to 30 minutes. UV curing requires less time and can generally be in the range of about two minutes or less. Curing around and along the substrate can be accomplished by incrementally or continuously using irradiation from multiple angles using spaced lamps and/or reflectors; rotation of the substrate, light source a light beam; longitudinal movement of the substrate, light source or light beam; or a combination of such techniques. The polymerizable composition is typically cured by irradiation with a suitable source of activating radiation such as ultraviolet (UV) radiation. Light sources may be narrow or broad spectrum or laser beam sources. Suitably the composition is cured using a high intensity broad spectrum ultraviolet lamp such as mercury arc capillary lamps which have some output in the UVC region (280 nm-100 nm). In some embodiments the composition is photocured with UV lamps that are sequenced or pulsed in a way that allows for some heat dissipation during the curing cycle.

In some embodiments the crosslinked coating thickness on the substrate is desirably in the range of from 0.1 micrometers or less to 20 micrometers or more (e.g., from 0.1 to 0.2 to 0.5 to 1 to 2 to 5 to 10 to 20 micrometers), more preferably, 0.1 to 5 micrometers. The coating thickness will be affected by the percent solids in the coating and the technique of application, among other factors. Multiple coatings may be applied to achieve a desired coating thickness.

As noted above, in some embodiments the coating compositions described herein may comprise a therapeutic agent, for example, selected from antimicrobial agents, antibiotic agents, anti-cancer agents, agents for treating calcifications, antirestenotic agents and antithrombotic agents, and combinations thereof, among others. The therapeutic agent(s) may be added to the coating composition prior to curing or applied onto the coating after it has been cured. Therapeutic agent(s) carried in the polymer coating may remain in the coating or elute out of the coating when the coating is wet, thereby delivering the therapeutic agent(s) to immediately adjacent areas of the body.

The invention is illustrated by the following non-limiting examples.

### Example 1. Impact of lower MW PVP on lubricity and durability (reference example).

Raw Materials:
- PVP K90, weight average molecular weight 900,000, International Specialty Products, Wayne, NJ, USA.
- PVP K30, weight average molecular weight 58,000, International Specialty Products
- PVP K15, weight average molecular weight 10,000, International Specialty Products
- Neopentyl glycol diacrylate (NPGDA), Sigma-Aldrich, St. Louis, MO, USA
- 2-Propanol (IPA), HPLC 99.5%, Sigma-Aldrich
- Reverse osmosis (RO) water

Over-the-wire catheters manufactured by Boston Scientific Corporation that had a distal shaft of Pebax™ 7033 were employed in these Examples. These catheters were exposed to a argon plasma in a commercial treatment system (March Plasma Systems, Concord, CA, USA) prior to coating. The coatings were applied and cured within a short time of plasma treatment.

A solvent mixture of water and IPA was prepared at 1 to 4 weight basis water to IPA. PVP (i.e., K90 100%, K90:K30 50:50 weight ratio, or K90:K15 50:50 weight ratio) and NPGDA were then added at a 4:1 PVP:NPGDA weight ratio to the solvent mixture to a total solids content of 5.5%.

Catheters were coated along the outer shaft up to the balloon using a sponge wipe coater with the respective coating compositions. Coated catheters were mounted in spaced straight parallel arrangement in fixtures that were then placed in a UV curing chamber and subjected to a curing protocol that applied UV energy to the surface of the rotating catheters so that the coating was cured around the circumference of the tubing.

Catheters were selected for lubricity and durability (L&D) testing. A catheter was cut into equal length pieces, the pieces submerged in water, weighed down with a given weight and subjected to reciprocal pulling and pushing cycles, measuring the frictional force (in grams) required to move the catheter piece initially (t=0) and through successive cycles. The coating thickness was determined on a central portion of the catheters. Average coating thicknesses for the coatings were in the range of about 0.5 to 1.5 micrometers.

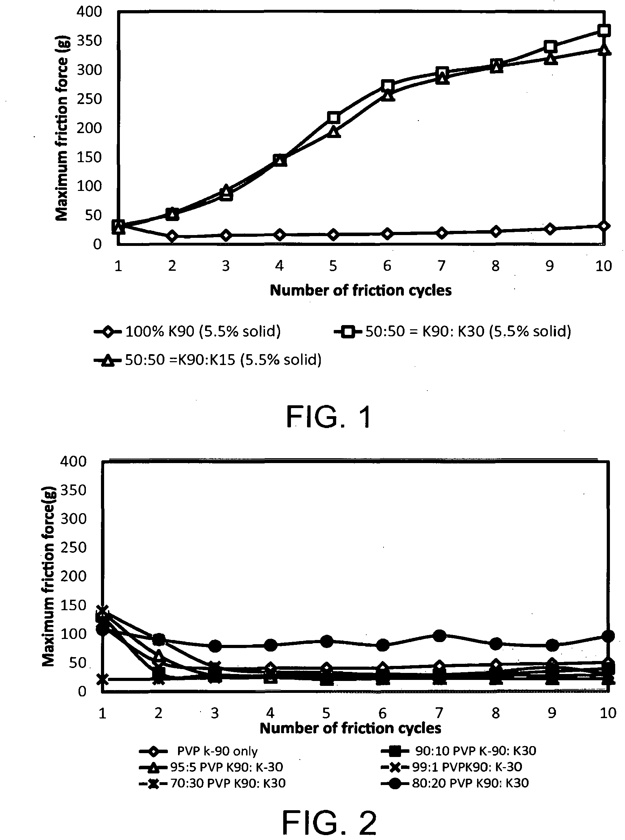

As seen from Fig. 1, the addition of lower MW PVP to the higher MW PVP in a 50:50 weight ratio improved lubricity, but resulted in less durability of the lubricity.

### Example 2. Impact of ratio of higher MW PVP to lower MW PVP on lubricity and durability.

Procedures analogous to that of Example 1 were followed with coating compositions of differing weight ratios of higher MW PVP to lower MW PVP. A solvent mixture of water and IPA was prepared at 1 to 4 weight basis water to IPA. PVP (100% K90, K90:K30 99:1 weight ratio, K90:K30 95:5 weight ratio, K90:K30 90:10 weight ratio, K90:K30 80:20 weight ratio or K90:K30 70:30 weight ratio) and NPGDA were then added at a 2:1 PVP:NPGDA weight ratio to the solvent mixture to a total solids content of 3.3%.

As can be seen from Fig. 2, lubricity and durability are acceptable over the entire range of higher MW PVP:lower MW PVP ratios, although the K90:K30 80:20 formulation exhibited slightly lower lubricity.

### Example 3. Impact of ratio of higher MW PVP to lower MW PVP on particulates.

Procedures analogous to that of Example 1 were followed with coating compositions of differing ratios of higher MW PVP to lower MW PVP. A solvent mixture of water and IPA was prepared at 1 to 4 weight basis water to IPA. PVP (100% K90, K90:K30 90:10 weight ratio, K90:K30 80:20 weight ratio, or K90:K30 70:30 weight ratio) and NPGDA were then added at a 2:1 PVP:NPGDA weight ratio to the solvent mixture to a total solids content of 3.3%.

For small particles, catheters were used for measurement of surface particulate count at t=0. The catheters were inserted into a tubular test apparatus with bends constructed to simulate features of clinical deployment. Water was circulated over the catheters for a fixed period of time. Particles larger than 10 micrometers that were transferred into the circulating water were counted using a laser particle counter. For large particles (>50 micron), solutions collected post laser count were filtered by using a 5um pore size filter, and the filter was subsequently analyzed by image analysis to obtain a particle size distribution.

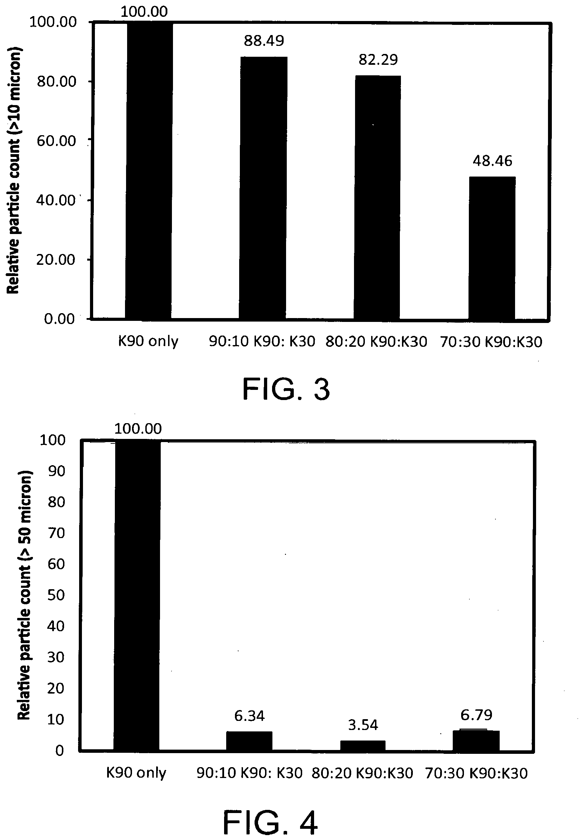

As can be seen from Figs. 3 and 4, small particle size counts were somewhat improved, and large particle counts were dramatically improved by the addition of lower MW PVP to the coating at all ratios.

Without wishing to be bound by theory, it is noted that lower MW PVP (i.e., K30) has been shown to be nearly 100% soluble (i.e., about 99%) when cured. The solubility of higher MW PVP (i.e., K90), on the other hand, has been shown to be much less soluble (i.e., about 33.5%) upon cure. Thus the higher MW PVP (i.e., K90) appears to be able to contribute to insoluble particles whereas the lower MW MVP (i.e., K30) cannot. Consequently, particulates can be significant reduced by replacing a portion of the higher MW PVP (i.e., K90) with lower MW MVP (i.e., K30).

### Example 4. Impact of solids content on lubricity and durability.

Procedures analogous to that of Example 1 were followed with coating compositions of differing solids content. A solvent mixture of water and IPA was prepared at 1 to 4 weight basis water to IPA. PVP (100% K90 or K90:K30 70:30 weight ratio) and NPGDA were then added at a 2:1 PVP:NPGDA weight ratio to the solvent mixture in varying total solids contents (2%, 3%, 3.3%, 4% or 5%).

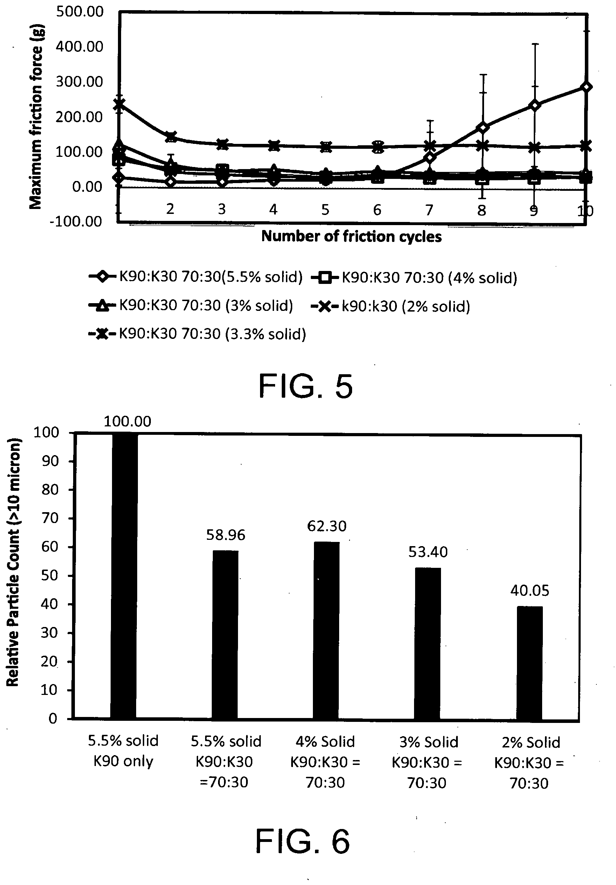

As seen from Fig. 5, good overall lubricity and durability performance was obtained for solids content ranging from 3% to 4%. The high solids content (5.5%) composition was found to lose durability after several passes. The low solids content (2%) composition was found to exhibit lower lubricity, possibly due to insufficient coating material.

### Example 5. Impact of solids content on particulates.

Procedures analogous to that of Example 2 were followed with coating compositions of differing solids content. A solvent mixture of water and IPA was prepared at 1 to 4 weight basis water to IPA. PVP (100 % K90 or K90:K30 70:30 weight ratio) and NPGDA were then added at a 2:1 PVP:NPGDA weight ratio to the solvent mixture at varying total solids contents (2%, 3%, 3.3%, 4% or 5%).

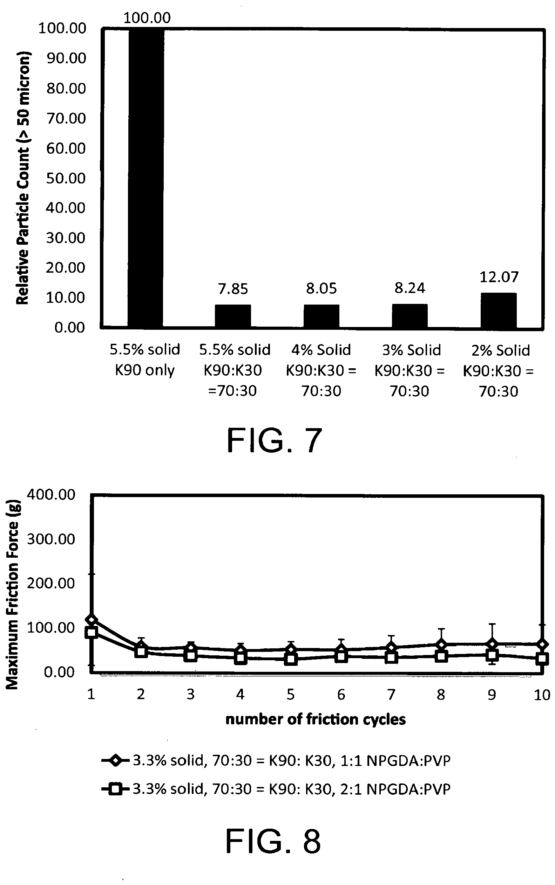

As can be seen from Figs. 6 and 7, no significant particle size trends were observed between 2% to 5.5% solid. Also, small particle size counts were significantly improved and large particle counts were dramatically improved by the addition of lower MW PVP to the coating.

### Example 6. Impact of ratio of PVP to NPGDA on lubricity and durability.

Procedures analogous to that of Example 1 were followed with coating compositions of differing ratios of PVP to NPGDA. A solvent mixture of water and IPA was prepared at 1 to 4 weight basis water to IPA. PVP (K90:K30 70:30 weight ratio) and NPGDA were then added to the solvent mixture at varying PVP:NPGDA weight ratios (2:1 to 1:1) to a total solids content of 3.3%.

As seen from Fig. 8, good overall lubricity and durability performance was obtained for PVP:NPGDA weight ratios ranging from 2:1 to 1:1.

### Example 7. Impact of ratio of PVP to NPGDA particulates.

Procedures analogous to that of Example 2 were followed with coating compositions of differing ratios of PVP to NPGDA. A solvent mixture of water and IPA was prepared at 1 to 4 weight basis water to IPA. PVP (100% K90 or K90:K30 70:30 weight ratio) and NPGDA were then added to the solvent mixture at varying PVP:NPGDA weight ratios (2:1 to 1:1) to a total solids content of 3.3%. The PVP:NPGDA weight ratio for 100% K90 was 2:1.

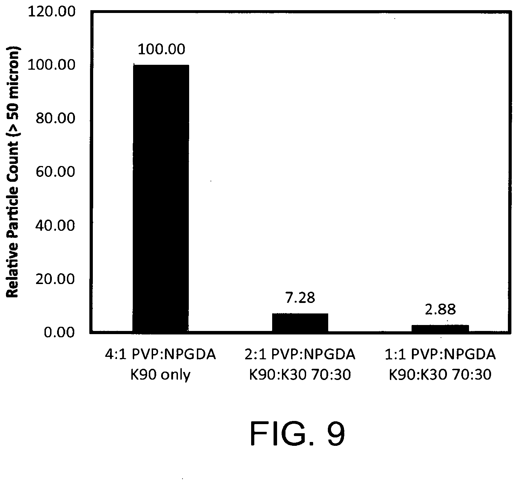

As seen from Fig. 9, no significant particle size trend was observed for K90:K30 mixtures (70:30 weight ratio) with PVP:NPGDA weight ratios ranging from 2:1 to 1:1. Large particle size counts were significantly improved by the addition of lower MW PVP to the coating.

## Claims

1. A lubricous coating composition comprising (a) a higher molecular weight polyvinylpyrrolidone, (b) a lower molecular weight polyvinylpyrrolidone, and (c) a polyfunctional unsaturated crosslinking agent, wherein the polyfunctional unsaturated crosslinking agent is a polyfunctional ethylenically unsaturated monomer, the weight ratio of higher molecular weight polyvinylpyrrolidone to lower molecular weight polyvinylpyrrolidone ranges from 70:30 to 90:10, and the weight ratio of total polyvinylpyrrolidone to polyfunctional ethylenically unsaturated monomer is in the range of 0.5:1 to 4:1.

2. The lubricous coating composition of claim 1, wherein the higher molecular weight polyvinylpyrrolidone has a K-value ranging from 85 to 95 and wherein the lower molecular weight polyvinylpyrrolidone has a K-value ranging from 15 to 32.

3. The lubricous coating composition of claim 1, wherein the crosslinking agent is a polyfunctional ethylenically unsaturated monomer selected from a di- or trifunctional acrylate compound, a di- or trifunctional methacrylate compound and a di- or trifunctional vinyl compound.

4. The lubricous coating composition of claim 3, wherein the crosslinking agent is neopentyl glycol diacrylate.

5. The lubricous coating composition of claim 1, further comprising a solvent.

6. The lubricous coating composition of claim 6, wherein the solvent is isopropanol in combination with water.

7. The lubricous coating composition of claim 6, wherein the weight ratio of water to isopropanol ranges from 50:50 to 10:90.

8. The lubricous coating composition of claim 1, further comprising a therapeutic agent which is preferably selected from antimicrobial agents, antibiotic agents, anti-cancer agents, agents for treating calcifications, antirestenotic agents, antithrombotic agents and combinations thereof.

9. A medical article comprising a medical article substrate that is at least partially covered by a layer of the lubricous coating composition in accordance with claim 1, wherein the polyfunctional unsaturated crosslinking agent is at least partially crosslinked.

10. The medical article of claim 9, wherein the layer ranges from 0.1 to 20 micrometers in thickness.

11. A method comprising applying the lubricous coating composition of claim 6 to a substrate in the form of a layer, removing at least a portion of the solvent, and crosslinking the composition by applying of UV light.

12. An article made by the method of claim 11, wherein the article is a medical article comprising a lubricious coating.

13. The medical article of claim 9 or claim 12, wherein the medical article is an implantable or insertable medical device.

## Patentansprüche

1. Eine gleitfähige Beschichtungszusammensetzung, umfassend (a) ein Polyvinylpyrrolidon mit höherem Molekulargewicht, (b) ein Polyvinylpyrrolidon mit niedrigerem Molekulargewicht und (c) ein polyfunktionelles ungesättigtes Vernetzungsmittel, wobei das polyfunktionelle ungesättigte Vernetzungsmittel ein polyfunktionelles ethylenisch ungesättigtes Monomer ist, das Gewichtsverhältnis von Polyvinylpyrrolidon mit höherem Molekulargewicht zu Polyvinylpyrrolidon mit niedrigerem Molekulargewicht im Bereich von 70:30 bis 90:10 liegt, und das Gewichtsverhältnis des gesamten Polyvinylpyrrolidons zum polyfunktionellen ethylenisch ungesättigten Monomer im Bereich von 0,5:1 bis 4:1 liegt.

2. Die gleitfähige Beschichtungszusammensetzung nach Anspruch 1, wobei das Polyvinylpyrrolidon mit höherem Molekulargewicht einen K-Wert im Bereich von 85 bis 95 aufweist und wobei das Polyvinylpyrrolidon mit niedrigerem Molekulargewicht einen K-Wert im Bereich von 15 bis 32 aufweist.

3. Die gleitfähige Beschichtungszusammensetzung nach Anspruch 1, wobei das Vernetzungsmittel ein polyfunktionelles ethylenisch ungesättigtes Monomer, ausgewählt aus einer di- oder trifunktionellen Acrylatverbindung, einer di- oder trifunktionellen Methacrylatverbindung und einer di- oder trifunktionellen Vinylverbindung, ist.

4. Die gleitfähige Beschichtungszusammensetzung nach Anspruch 3, wobei das Vernetzungsmittel Neopentylglycoldiacrylat ist.

5. Die gleitfähige Beschichtungszusammensetzung nach Anspruch 1, weiter umfassend ein Lösungsmittel.

6. Die gleitfähige Beschichtungszusammensetzung nach Anspruch 6, wobei das Lösungsmittel Isopropanol in Kombination mit Wasser ist.

7. Die gleitfähige Beschichtungszusammensetzung nach Anspruch 6, wobei das Gewichtsverhältnis von Wasser zu Isopropanol im Bereich von 50:50 bis 10:90 liegt.

8. Die gleitfähige Beschichtungszusammensetzung nach Anspruch 1, weiter umfassend ein Therapeutikum, welches vorzugsweise aus antimikrobiellen Mitteln, Antibiotika, Mitteln gegen Krebs, Mitteln zur Behandlung von Verkalkungen, Antirestenosemitteln, Antithrombotika und Kombinationen davon ausgewählt ist.

9. Ein medizinischer Gegenstand, umfassend ein Trägermaterial eines medizinischen Gegenstands, das zumindest teilweise von einer Schicht der gleitfähigen Beschichtungszusammensetzung nach Anspruch 1 bedeckt ist, wobei das polyfunktionelle ungesättigte Vernetzungsmittel zumindest teilweise vernetzt ist.

10. Der medizinische Gegenstand nach Anspruch 9, wobei die Schicht eine Dicke im Bereich von 0,1 bis 20 Mikrometern hat.

11. Ein Verfahren, umfassend Aufbringen der gleitfähigen Beschichtungszusammensetzung nach Anspruch 6 auf ein Substrat in Form einer Schicht, Entfernen von zumindest einem Teil des Lösungsmittels und Vernetzen der Zusammensetzung unter Anwendung von UV-Licht.

12. Ein Gegenstand, hergestellt durch das Verfahren nach Anspruch 11, wobei der Gegenstand ein medizinischer Gegenstand ist, der eine gleitfähige Beschichtung umfasst.

13. Der medizinische Gegenstand nach Anspruch 9 oder Anspruch 12, wobei der medizinische Gegenstand eine implantierbare oder einführbare medizinische Vorrichtung ist.

## Revendications

1. Composition de revêtement lubrifiant comprenant (a) une polyvinylpyrrolidone de masse moléculaire élevée, (b) une polyvinylpyrrolidone de faible masse moléculaire, et (c) un agent de réticulation insaturé polyfonctionnel, où l'agent de réticulation insaturé polyfonctionnel est un monomère éthyléniquement insaturé polyfonctionnel, le rapport massique de polyvinylpyrrolidone de masse moléculaire élevée à polyvinylpyrrolidone de faible masse moléculaire est de 70:30 à 90:10, et le rapport massique du polyvinylpyrrolidone totale au monomère éthyléniquement insaturé polyfonctionnel est de 0,5 :1 à 4 :1.

2. Composition de revêtement lubrifiant selon la revendication 1, où la polyvinylpyrrolidone de masse moléculaire élevée présente une valeur K de 85 à 95 et où la polyvinylpyrrolidone de faible masse moléculaire présente une valeur K de 15 à 32.

3. Composition de revêtement lubrifiant selon la revendication 1, où l'agent de réticulation est un monomère éthyléniquement insaturé polyfonctionnel choisi parmi un composé d'acrylate di- ou trifonctionnel, un composé de méthacrylate di- ou trifonctionnel et un composé de vinyle di- ou trifonctionnel.

4. Composition de revêtement lubrifiant selon la revendication 3, où l'agent de réticulation est le diacrylate de néopentyl glycol.

5. Composition de revêtement lubrifiant selon la revendication 1, comprenant de plus un solvant.

6. Composition de revêtement lubrifiant selon la revendication 6, où le solvant est l'isopropanol en combinaison avec de l'eau.

7. Composition de revêtement lubrifiant selon la revendication 6, où le rapport massique d'eau à isopropanol est de 50:50 à 10:90.

8. Composition de revêtement lubrifiant selon la revendication 1, comprenant de plus un agent thérapeutique qui est de préférence choisi parmi des agents antimicrobiens, des agents antibiotiques, des agent anti-cancer, des agents pour traiter des calcifications, des agents anti-resténotiques, des agents antithrombotiques et des combinaisons de ceux-ci.

9. Article médical comprenant un substrat d'article médical qui est au moins partiellement recouvert par une couche de la composition de revêtement lubrifiant selon la revendication 1, où l'agent de réticulation insaturé polyfonctionnel est au moins partiellement réticulé.

10. Article médical selon la revendication 9, où la couche est de 0,1 à 20 micromètres d'épaisseur.

11. Procédé comprenant l'application de la composition de revêtement lubrifiant selon la revendication 6 sur un substrat dans la forme d'une couche, l'élimination d'au moins une portion du solvant, et la réticulation de la composition par application d'une lumière UV.

12. Article fabriqué par le procédé selon la revendication 11, où l'article est un article médical comprenant un revêtement lubrifiant.

13. Article médical selon la revendication 9 ou la revendication 12, où l'article médical est un dispositif médical implantable ou insérable.
